# EUROPEAN PATENT SPECIFICATION

(11) **EP 0 587 645 B1**
(45) Date of publication and mention of the grant of the patent: **27.02.2002**
(21) Application number: 92911170.6
(22) Date of filing: 11.06.1992
(51) Int. Cl.: A61K 31/352, A61K 31/436

(54) **BENZO- AND PYRIDOPYRAN DERIVATIVES HAVING ANXIOLYTIC AND ANTI-CONVULSANT ACTIVITY**
BENZO- UND PYRIDOPYRANDERIVATE MIT ANXIOLYTISCHER UND ANTIKONVULSIVER AKTIVITÄT
DERIVES DE BENZO- ET PYRIDOPYRANE PRESENTANT UNE ACTIVITE ANXIOLYTIQUE ET ANTICONVULSIVE

(30) Priority: 13.06.1991 GB 9112721
(43) Date of publication of application: 23.03.1994
(60) Divisional application: 01200951.0
(73) Proprietor: SMITHKLINE BEECHAM PLC, Brentford, Middlesex TW8 9EP (GB)
(72) Inventor: EVANS, J. M., SmithKline Beecham Pharmaceuticals, Essex CM19 5AD (GB); THOMPSON, M., SmithKline Beecham Pharmaceuticals, Essex CM19 5AD (GB); UPTON, Neil, SmithKline Beecham Pharmaceuticals, Essex CM19 5AD (GB)
(74) Representative: Valentine, Jill Barbara
(86) International application number: GB9201045
(87) International publication number: WO9222293

(56) References cited:
- EP-A- 0 126 311
- EP-A- 0 205 292
- EP-A- 0 250 077
- EP-A- 0 370 901
- US-A- 4 334 067

## Description

European Published Patent Application No. 0126311 discloses substituted benzopyran compounds having blood pressure lowering activity, including 6-acetyl-trans-4-(4-fluorobenzoylamino)-3,4-dihydro-2,2-dimethyl-2H-1-benzopyran-3-ol.

Also EP-A-0 376 524, EP-A-0 205 292, EP-A-0 250 077, EP-A-0 093 535, EP-A-0 150 202, EP-A-0 076 075 and WO/89/05808 (Beecham Group plc) describe certain benzopyran derivatives which possess anti-hypertensive activity.

EP-A-0 350 805 (Biersdorf), EP-A-0 277 611, EP-A-0 277612, EP-A-0 337 179 and EP-A-0 355 565 (Hoechst Aktiengesellschaft); and EP-A-0 466 131 (Nissan Chemical Industries Ltd) also describe certain benzopyran derivatives which are believed to possess anti-hypertensive activity.

EP-A-0 430 621 and EP-A-0 385 584 (Beecham Group plc) describe the resolution of certain intermediates useful in the preparation of the compounds described in the above mentioned patent applications.

EP-A-0 194 884 (E. Lilly) describes certain amino substituted benzopyran derivatives possessing anti-convulsant activity.

US 4,048,317 describes certain flavan compounds which may be used as pharmaceutical agents having anti-convulsive, anti-ulcer, anti-arrhythmic and diuretic activities.

It has now been surprisingly found that certain compounds of formula (I) possess anxiolytic and anti-convulsant activity, and are also believed to have utility in the treatment or prevention of mania, depression and the effects associated with withdrawal from substances of abuse.

Compounds of formula (I) may therefore be used for the preparation of medicaments for the treatment and/or prophylaxis of disorders treatable and/or preventable with anti-convulsive agents, such as epilepsy.

Accordingly, the present invention provides the use of a compound of formula (I) or pharmaceutically acceptable salt thereof: 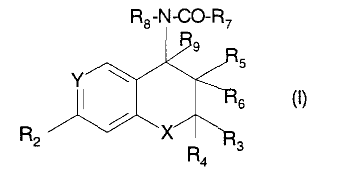 wherein:
either Y is N and R₂ is hydrogen, or Y is C-R₁
where:
either one of R₁ and R₂ is hydrogen and the other is selected from hydrogen, C₃₋₈ cycloalkyl, C₁₋₆ alkyl optionally interupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is -CF₂-, -CO-, -CH₂- or CH(OH), trifluoromethoxy, C₁₋₆ alkylsulphinyl, C₁₋₆ alkylsulphonyl, C₁₋₆ alkoxysulphinyl, C₁₋₆ alkoxysulphonyl, aryl, heteroaryl, arylcarbonyl, heteroarylcarbonyl, arylsulphinyl, heteroarylsulphinyl, arylsulphonyl, heteroarylsulphonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, any amino moiety being optionally substituted by one or two C₁₋₆ alkyl groups, or C₁₋₆ alkylsulphinylamino, C₁₋₆ alkylsulphonylamino, C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl;
one of R₃ and R₄ is hydrogen or C₁₋₄ alkyl and the other is C₁₋₄ alkyl or R₃ and R₄ together are C₂₋₅ polymethylene;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ is hydrogen or C₁₋₂ alkyl and R₉ is hydrogen;
R₇ is fluorophenyl;
R₈ is hydrogen or C₁₋₆ alkyl;
the R₈-N-CO-R₇ group being trans to the R₅ group;
and X is oxygen or NR₁₀ where R₁₀ is hydrogen or C₁₋₆ alkyl;
wherein aryl means phenyl or naphthyl and heteroaryl means a 5- or 6-membered monocyclic or 9- or 10-membered bicyclic group containing up to three heteroatoms which are the same or different and are selected from oxygen, nitrogen and sulphur; and each aryl or heteroaryl group is optionally substituted with up to three sustituents independently selected from the group consisting of C₁₋₄ alkyl, C₁₋₄ alkoxy, halo, hydroxy, nitro, cyano and SOₙH where n=0 to 2;
for the manufacture of a medicament for the treatment and/or prophylaxis of disorders treatable or preventable with anti-convulsive agents.

Compounds of formula (I) include those in which Y is C-R₁, where either one of R₁ and R₂ is hydrogen and the other is selected from hydrogen, C₁₋₆ alkylcarbonyl, C₁₋₆ alkoxycarbonyl, C₁₋₆ alkylcarbonyloxy, C₁₋₆ alkylhydroxymethyl, nitro, cyano, chloro, trifluoromethyl, trifluoromethoxy, C₁₋₆ alkylsulphinyl, C₁₋₆ alkylsulphonyl, C₁₋₆ alkoxysulphinyl, C₁₋₆ alkoxysulphonyl, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, the amino moiety being optionally substituted by one or two C₁₋₆ alkyl groups, or C₁₋₆ alkylsulphinylamino, C₁₋₆ alkylsulphonylamino,C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl;
one of R₃ and R₄ is hydrogen or C₁₋₄ alkyl and the other is C₁₋₄ alkyl or R₃ and R₄ together are C₂₋₅ polymethylene;
R₅ is hydroxy and R₆ is hydrogen;
R₇ is fluorophenyl;
R₈ is hydrogen or C₁₋₆ alkyl; and
R₉ is hydrogen;
the R₈-N-CO-R₇ group being trans to the R₅ group.

All C₁₋₆ alkyl or alkyl containing groups in formula (I) are preferably selected from methyl, ethyl, n- and iso-propyl, n-, iso-, sec- and tert-butyl.

Suitable C₃₋₈ cycloalkyl groups include cyclopropyl, cyclobutyl, cyclopentyl, cyclohexyl, cycloheptyl and cyclooctyl.

Heteroaryl includes a 5- or 6- membered monocyclic or 9- or 10- membered bicyclic of which 5- or 6- membered monocyclic heteroaryl is preferred. In addition, 5- or 6-membered monocyclic or 9- or 10-membered bicyclic heteroaryl preferably contains one, two or three heteroatoms which are selected from oxygen, nitrogen and sulphur and which, in the case of there being more than one heteroatom, are the same or different. Examples of 5- or 6-membered monocyclic heteroaryl containing one, two or three heteroatoms which are selected from oxygen, nitrogen and sulphur include furyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2-and 3-furyl, 2- and 3-pyrryl, 2- and 3-thienyl, and 2-, 3- and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazolyl. Preferred examples of such groups include 2- and 3-benzofuryl, 2- and 3-benzothienyl, and 2- and 3-indolyl, and 2- and 3-quinolyl.

Suitable examples of groups or atoms for optional substitution of aryl and heteroaryl include one, two or three substituents independently selected from C₁₋₄ alkyl, C₁₋₄ alkoxy, halo (such as fluoro, chloro, bromo), hydroxy, nitro,cyano and SOₙH, where n=0 to 2.

Preferably R₂ is hydrogen.

Examples of suitable R₁ substituents include cyano, methoxy, trifluoromethoxy, chloro, trifluoromethyl, ethylcarbonyl, acetyl, hydrogen, methyl, ethyl, iso-propyl,tertiary-butyl, nitro, C₂F₅, methoxycarbonyl, phenylsulphonyl, phenyl, fluoro, iodo, cyclopentyl, aminocarbonylmethyl and 1-hydroxyethyl. Preferably R₁ is cyano, acetyl or ethyl.

Preferably R₃ and R₄ are both methyl.

Preferably R₅ is hydroxy and R₆ and R₉ are hydrogen or R₅ is hydroxy, R₆ is C₁₋₂ alkyl and R₉ is hydrogen, more preferably R₅ is hydroxy and R₆ and R₉ are hydrogen.

It should be appreciated that the term fluorophenyl relating to R₇ encompasses phenyl which has 1,2,3,4 or 5 fluoro groups attached to the phenyl ring. Preferably there are 1 or 2 fluoro groups attached to the phenyl ring and most preferably there is 1 fluoro group attached to the ring.

The fluoro group or groups may be in any position around the phenyl ring, preferably in the case of mono fluorophenyl the fluoro is in the 3 or 4 position.

In the case of difluorophenyl, preferably the fluoro substituents are at positions 2,4 or 3,4.

Preferably R₈ is hydrogen or C₁₋₄ alkyl, more preferably R₈ is hydrogen, methyl or ethyl.

Preferably X is oxygen.

It should be appreciated that the compounds of formula (I) may have chiral carbon atoms at positions 2, 3 and 4 and therefore may exist as enantiomers. The present invention extends to each enantiomer and to mixtures thereof including racemates. Preferably the compounds of formula (I) exist as 4S, 3R enantiomers.

It should also be appreciated that certain R₁ substituents also have chiral centres and therefore may exist as enantiomers. The present invention extends to each enantiomer and to mixtures thereof including racemates.

An example of a compound of formula (I) is trans-6-acetyl-4-(4-fluorobenzoylamino)-3,4-dihydro-2,2-dimethyl-2H-1-benzopyran-3-ol (compound X).

Examples of compounds of formula (I) suitable for use in the present invention are:
trans-6-Cyano-3,4-dihydro-2,2-dimethyl-4-(4-fluorobenzoylamino)2H-1-benzopyran-3-ol, (Example 5),
trans-6-Chloro-3,4-dihydro-2,2-dimethyl-4-(4-fluorobenzoylamino)2H-1-benzopyran-3-ol, (Example 6),
trans-6-Trifluoromethyl-4-(4-fluorobenzoylamino)-3,4-dihydro-2,2-dimethyl-2H-1-benzopyran-3-ol, (Example 7),
trans-6-Cyano-4-(4-fluorobenzoylmethylamino)-3,4-dihydro- 2,2-dimethyl-2H-1-benzopyran-3-ol, (Example 8),
trans-6-Ethylcarbonyl-3,4-dihydro-2,2-dimethyl-4-(4-fluorobenzoylamino)2H-1-benzopyran-3-ol, (Example 9),
trans-6-Ethylcarbonyl-3,4-dihydro-2,2-dimethyl-4-(4-fluorobenzoylmethylamino)2H-1-benzopyran-3-ol, (Example 10),
trans-6-Acetyl-4-(4-fluorobenzoylmethylamino)-3,4-dihydro-2,2-dimethyl-2H-1-benzopyran-3-ol, (Example 11),
trans-6-Cyano-3,4-dihydro-4-(4-fluorobenzoylethylamino)-2,2-dimethyl-2H-1-benzopyran-3-ol (Example 12),
trans-4-(4-Fluorobenzoylamino)-3,4-dihydro-2,2-dimethyl-7-nitro-2H-1-benzopyran-3-ol (Example 13),
trans-6-Acetyl-4-(4-Fluorobenzoylamino)-3,4-dihydro-2,2-dimethyl-2H-1-benzopyran-3-ol (Example 18),
trans-6-Acetyl-4R-(4-fluorobenzoylamino)-3,4-dihydro-2,2-dimethyl-2H-1-benzopyran-3S-ol (Example 19),
trans-6-Acetyl-4S-(4-fluorobenzoylamino)-3,4-dihydro-2,2-dimethyl-2H-1-benzopyran-3R-ol (Example 20),
trans-6-Cyano-4S-(4-fluorobenzoylamino)-3,4-dihydro-2,2-dimethyl-2H-1-benzopyran-3R-ol (Example 25),
trans-6-Cyano-4-(4-fluorobenzoylamino)-3,4-dihydro-2,2,3-trimethyl-2H-1-benzopyran-3-ol (Example 26),
trans-4-(4-Fluorobenzoylamino)-3,4-dihydro-2,2-dimethyl-6-nitro-2H-1-benzopyran-3-ol (Example 27),
trans-4-(4-Fluorobenzoylamino)-3,4-dihydro-6-methoxycarbonyl-2,2-dimethyl-2H-1-benzopyran-3-ol (Example 30),
trans-6-Fluoro-4-(4-fluorobenzoylamino)-3,4-dihydro-2,2-dimethyl-2H-1-benzopyran-3-ol (Example 34),
trans-4-(4-fluorobenzoyl-methylamino)-6-trifluoromethyl-3,4-dihydro-2,2-dimethyl-2H-1-benzopyran-3-ol (Example 42),
trans-4-(4-Fluorobenzoylamino)-3,4-dihydro-6-iodo-2,2-dimethyl-2H-1-benzopyran-3-ol (Example 47) and
trans- 6-Aminocarbonylmethyl-4-(4-fluorobenzoylamino)-3,4-dihydro-2,2-dimethyl-2H-1-benzopyran-3-ol (Example 49).

The administration to the mammal may be by way of oral or parenteral administration.

An amount effective to treat the disorders hereinbefore described depends on the usual factors such as the nature and severity of the disorders being treated and the weight of the mammal. However, a unit dose will normally contain 1 to 1000 mg, suitably 1 to 500 mg, for example an amount in the range of from 2 to 100 mg such as 2, 5, 10, 20, 30, 40, 50 and 100 mg of the active compound. Unit doses will normally be administered once or more than once per day, for example 2, 3, 4, 5 or 6 times a day, more usually 2 to 4 times a day, such that the total daily dose is normally in the range, for a 70 kg adult of 1 to 1000 mg, for example 50 to 500 mg, that is in the range of approximately 0.01 to 50 mg/kg/day, more usually 0.1 to 50 mg/kg/day, for example 1 to 50 mg/kg/day.

It is greatly preferred that the compound of formula (I) is administered in the form of a unit-dose composition, such as a unit dose oral, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing the compound and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

As is common practice, the compositions will usually be accompanied by written or printed directions for use in the medical treatment concerned.

Accordingly, a pharmaceutical composition for use in the treatment and/or prophylaxis of disorders treatable or preventable with anti-convulsive agents, such as epilepsy, may comprise a compound of formula (I), or a pharmaceutically acceptable salt thereof, in particular, compounds of examples 1 to 50, and a pharmaceutically acceptable carrier.

Such compositions may be prepared in the manner as hereinbefore described.

Compounds of formula (I) suitable for use in the present invention include the group of compounds in which R₇ is 2- or 3-fluorophenyl, 2, 4- or 3,4- difluorophenyl. Such compounds will hereinafter be referred to as compounds of formula (Ia).

Examples of compounds of formula (Ia) include:
trans-6-Cyano-3,4-dihydro-2,2-dimethyl-4-(3-fluorobenzoylamino)2H-1-benzopyran-3-ol (Example 1),
trans-6-Cyano-3,4-dihydro-2,2-dimethyl-4-(2-fluorobenzoylamino)2H-1-benzopyran-3-ol (Example 2),
trans-6-Trifluoromethoxy-3,4-dihydro-2,2-dimethyl-4-(3- fluorobenzoylamino)2H-1-benzopyran-3-ol (Example 3),
trans-6-Cyano-4S-(3-fluorobenzoylamino)-3,4-dihydro-2,2-dimethyl-2H-1-benzopyran-3R-ol (Example 21),
trans-6-Cyano-4R-(3-fluorobenzoylamino)-3,4-dihydro-2,2-dimethyl-2H-1-benzopyran-3S-ol (Example 22),
trans-6-Cyano-4S-(2-fluorobenzoylamino)-3,4-dihydro-2,2-dimethyl-2H-1-benzopyran-3R-ol (Example 24),
trans-6-Cyano-4S-(2,4-difluorobenzoylamino)-3,4-dihydro-2,2-dimethyl-2H-1-benzopyran-3R-ol (Example 28),
trans-6-Acetyl-4-(3-fluorobenzoylamino)-3,4-dihydro-2,2-dimethyl-2H-1-benzopyran-3-ol (Example 31),
trans-4-(2-Fluorobenzoylamino)-3,4-dihydro-2,2-dimethyl-2H-pyrano[3,2-c]pyridin-3-ol (Example 38),
trans-4-(3-Fluorobenzoylamino)-3,4-dihydro-2,2-dimethyl-2H-pyrano[3,2-c]pyridin-3-ol (Example 39),
trans-6-Cyano-4-(3,4-difluorobenzoyl-methylamino)-3,4-dihydro-2,2-dimethyl-2H-1-benzopyran-3-ol (Example 41) and
trans-6-cyano-4S-(3,4-difluorobenzoylamino)-3,4-dihydro-2,2-dimethyl-2H-1-benzopyran-3R-ol (Example 44).

Compounds of formula (I) suitable for use in the present invention also include the group of compounds in which Y is CR₁ where R₁ and R₂ are both hydrogen or one of R₁ and R₂ is trifluoromethoxy, C₁₋₆ alkyl interrupted with oxygen or substituted with hydroxy, C₁₋₆ alkoxy or substituted amino-carbonyl, CF₃A-(where A is -CF₂-, -CO-, -CH₂) or CH(OH)), aryl sulphonyl, aryl C₃₋₈ cycloalkyl, C₁₋₆ alkoxy, heteroaryl, arylcarbonyl, heteroarylcarbonyl, arylsulphinyl, heteroarylsulphinyl, heteroarylsulphonyl, in which any aromatic moiety is optionally substituted and the other is hydrogen. Such compounds will hereinafter be referred to as compounds of formula (Ib).

Examples of compounds of formula (Ib) include:
trans-3,4-Dihydro-2,2-dimethyl-4-(4-fluorobenzoylamino)-2H-1-benzopyran-3-ol (Example 4),
trans-4-(4-Fluorobenzoylamino)-3,4-dihydro-2,2,6-trimethyl-2H-1-benzopyran-3-ol (Example 14),
trans-6-Ethyl-4-(4-fluorobenzoylamino)-3,4-dihydro-2,2-dimethyl-2H-1-benzopyran-3-ol (Example 15),
trans-6-Ethyl-4-(4-fluorobenzoylethylamino)-3,4-dihydro-2,2-dimethyl-2H-1-benzopyran-3-ol (Example 16),
trans-4-(4-Fluorobenzoylamino)-3,4-dihydro-2,2-dimethyl-6-isopropyl-2H-1-benzopyran-3-ol (Example 17),
trans-6-Ethyl-4-(4-fluorobenzoylmethylamino)-3,4-dihydro-2,2-dimethyl-2H-1-benzopyran-3-ol (Example 23),
trans-4-(4-Fluorobenzoylamino)-6-pentafluoroethyl-3,4-dihydro-2,2-dimethyl-2H-1-benzopyran-3-ol (Example 29),
trans-4-(4-Fluorobenzoylamino)-3,4-dihydro-2,2-dimethyl-6-phenylsulphonyl-2H-1-benzopyran-3-ol (Example 32),
trans-4-(4-Fluorobenzoylamino)-3,4-dihydro-2,2-dimethyl-6-phenyl-2H-1-benzopyran-3-ol (Example 33),
trans-6-Ethyl-4S-(4-fluorobenzoylamino)-3,4-dihydro-2,2-dimethyl-2H-1-benzopyran-3R-ol (Example 35),
trans-4R-(4-Fluorobenzoylamino)-3,4-dihydro-6-(1-hydroxyethyl)-2,2-dimethyl-2H-1-benzopyran-3S-ol (Example 36),
trans-4S-(4-Fluorobenzoylamino)-3,4-dihydro-6-(1-hydroxyethyl)-2,2-dimethyl-2H-1-benzopyran-3R-ol (Example 37),
trans-4-(4-Fluorobenzoyl-methylamino)-3,4-dihydro-2,2-dimethyl-2H-1-benzopyran-3-ol (Example 43),
trans-6-t-Butyl-4-(4-fluorobenzoylamino)-3,4-dihydro-2,2-dimethyl-2H-1-benzopyran-3-ol (Example 46),
trans-4-(4-Fluorobenzoylamino)-3,4-dihydro-2,2-dimethyl-6-cyclopentyl-2H-1-benzopyran-3-ol (Example 48) and
trans- 4-(4-Fluorobenzoylamino)-3,4-dihydro-6-methoxy-2,2-dimethyl-2H-1-benzopyran-3-ol (Example 50).

Compounds of formula (I) include compounds in which Y is N and R₂ is hydrogen. Examples of compounds of formula (I) in which Y is N and R₂ is hydrogen include:
trans-4-(2-Fluorobenzoylamino)-3,4-dihydro-2,2-dimethyl-2H-pyrano[3,2-c]pyridin-3-ol (Example 38),
trans-4-(3-Fluorobenzoylamino)-3,4-dihydro-2,2-dimethyl-2H-pyrano[3,2-c]pyridin-3-ol (Example 39) and
trans-4-(4-Fluorobenzoylamino)-3,4-dihydro-2,2-dimethyl-2H-pyrano[3,2-c]pyridin-3-ol (Example 40).

Compounds of formula (I) also include compounds in which X is NR₁₀ where R₁₀ is hydrogen or C₁₋₆ alkyl. An example of such a compound is:
trans-6-Cyano-4-(4-fluorobenzoylamino)-2,2-dimethyl-1,2,3,4-tetrahydroquinolin-3-ol (Example 45).

Generally, compounds of formula (I) may be prepared by procedures generally described or analogous to those described in EP-0126311, EP-0376524, EP-205292, EP-0250077, EP-0093535,
EP-0150202, EP-0076075, WO/89/05808, EP-0350805, EP-0277611,
EP-0277612, EP-0337179, EP-0355565 and EP-0466131.

Those compounds of formula (I) which are hereinafter illustrated in Examples 5-13, 18-20, 25-27, 30, 34, 42, 47 and 49, or pharmaceutically acceptable salts thereof, may be prepared by a process which comprises acylating a compound of formula (II'): 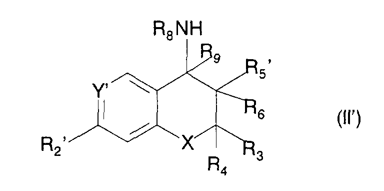 wherein Y', R₂' and R₅' are the required variables Y, R₂ or R₅ as defined in formula (I) or a group convertible thereto and R₃, R₄, R₆, R₈, R₉ and X are the required variables as defined in formula (I), the R₈NH group being trans to the R₅ group, with an acylating agent of formula (IIIb):

R₇COL, (IIIb)

where R₇ is as required and as defined in formula (I) and L₁ is a leaving group; thereafter optionally or as necessary and in any appropriate order converting any R₁', R₂' and R₅' groups to R₁, R₂ and R₅ respectively, interconverting R₈ when hydrogen to C₁₋₆ alkyl, separating any enantiomers and forming a pharmaceutically acceptable salt or solvate.

Compounds of formula (Ia), or a pharmaceutically acceptable salt thereof, may be prepared by a process which comprises acylating a compound of formula (IIa): 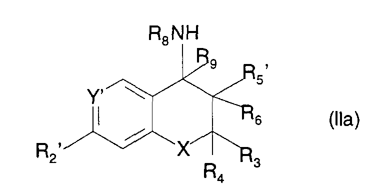 wherein Y', R₂' and R₅' are Y, R₂ or R₅ as defined in formula (I) or a group convertible thereto and R₃, R₄, R₆, R₈, R₉ and X are as defined in formula (I), the R₈NH group being trans to the R₅' group, with an acylating agent of formula (IIIa):

R₇^{a}COL₁ (IIIa)

where R₇a is 2- or 3-fluorophenyl or 2,4- or 3,4- difluorophenyl and L₁ is a leaving group; thereafter optionally or as necessary and in any appropriate order converting any Y', R₂' or R₅' group to Y, R₂ or R₅ respectively, interconverting R₈ when hydrogen to C₁₋₆ alkyl, separating any enantioners and forming a pharmaceutically acceptable salt or solvate.

Compounds of formula (Ib), or a pharmaceutically acceptable salt thereof, may be prepared by a process which comprises acylating a compound of formula (IIb): 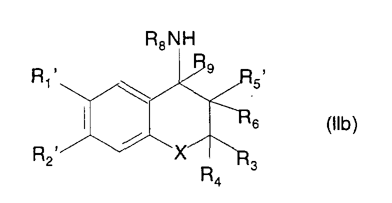 where R₁' and R₂' are both hydrogen or one of R₁ and R₂ is trifluoromethoxy, C₁₋₆ alkyl optionally interrupted with oxygen or substituted with hydroxy, C₁₋₆ alkoxy or substituted aminocarbonyl, CF₃A- (where A is CF₂,-CO-,-CH₂- or CH(OH)), aryl, aryl sulphonyl, aryl C₃₋₈ cycloalkyl, C₁₋₆ alkoxy, heteroaryl, arylcarbonyl, heteroarylcarbonyl, arylsulphinyl, heteroarylsulphinyl, heteroarylsulphonyl, in which any aromatic moiety is optionally substituted and the other is hydrogen, or groups convertible to any of these; R₅' is R₅ as defined in formula (I) or a group convertible thereto and R₃, R₄, R₆, R₈, R₉ and X are as defined in formula (I) the R₈NH group being trans to the R₅' group, with a compound of formula (IIIb):

R₇COL₁ (IIIb)

where R₇ is as defined in formula (I) and L₁ is a leaving group; thereafter optionally or as necessary and in any appropriate order converting any R₁', R₂' and R₅' groups to R₁, R₂ and R₅ respectively, interconverting R₈ when hydrogen to C₁₋₆ alkyl, separating any enantiomers and forming a pharmaceutically acceptable salt or solvate.

Compounds of formula (I) in which Y is N and R₂ is hydrogen, or a pharmaceutically acceptable salt thereof, may be prepared by a process which comprises acylating a compound of formula (IIc): 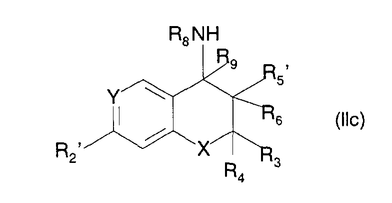 in which Y is N, R₅' is R₅, as defined in relation to formula (I) or a group convertible to R₅ and R₂' is hydrogen or a group convertible thereto, R₆, R₃, R₄, R₈, R₉ and X are as defined in formula (I), the R₈NH group being trans to the R₅' group, with an acylating agent of formula (IIIb):

R₇COL₁ (IIIb)

where R₇ is as defined in formula (I) and L₁ is a leaving group; thereafter optionally or as necessary and in any appropriate order converting any R₂' or R₅' group to hydrogen or R₅ respectively, interconverting R₈ when hydrogen to C₁₋₆ alkyl, separating any enantiomers and forming a pharmaceutically acceptable salt or solvate.

Compounds of formula (I) in which X is NR₁₀ where R₁₀ is hydrogen or C₁₋₆ alkyl, or a pharmaceutically acceptable salt thereof, may be prepared by a process which comprises acylating a compound of formula (IId): 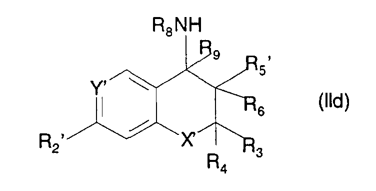 in which X' is NR₁₀' where R₁₀' is hydrogen or C₁₋₆ alkyl or a group convertible thereto, Y', R₂' and R₅ are Y, R₂ and R₅ respectively as defined in formula (I) or groups convertible thereto and R₃, R₄, R₆, R₈ and R₉ are as defined in formula (I), the R₈NH group being trans to the R₅' group, with an acylating agent of formula (IIIb):

R₇COL₁ (IIIb)

where R₇ is as defined in formula (I) and L₁ is a leaving group, thereafter optionally or as necessary and in any appropriate order converting any Y', R₁₀', R₂' or R₅' group to Y, R₁₀, R₂ or R₅ respectively, interconverting R₈ when hydrogen to C₁₋₆ alkyl, separating any enantiomers and forming a pharmaceutically acceptable salt or solvate.

Examples of suitable leaving groups L₁ include those mentioned in the above-mentioned patents, in particular EP-A-0 126 311 or are conventional in the art.

The reaction conditions which may be used to carry out the above reactions are as outlined or analogous to those described in the above-mentioned patents, in particular EP-A-0 126 311.

In particular, the leaving group (L₁) is a group that is displaceable by a primary or secondary amino nucleophile. Examples of such a group include C₁₋₄ alkylcarbonyloxy and halogen, such as chloro and bromo. When the leaving group (L₁) is any of these examples, the acylating agent of formula (IIIa) or (IIIb) is either an acid anhydride or an acid halide. When it is an acid anhydride, it is, preferably, a mixed anhydride, which may be prepared in situ from an aromatic or heteroaromatic carboxylic acid and an alkyl chlorocarbonate, such as ethyl chloroformate.

When the acylating agent of formula (IIIa) or (IIIb) is an acid anhydride, the acylation of the compound formula (IIa), (IIb), (IIc) or (IId) is, preferably carried out using the anhydride as the solvent in the presence of an acid acceptor, such as sodium acetate.

When the acylating agent of formula (IIIa) or (IIIb) is an acid halide, the acylation of the compound of formula (IIa), (IIb), (IIc) or (IId), is, preferably, carried out in a non-aqueous medium, such as methylene chloride, in the presence of an acid acceptor, such as triethylamine, trimethylamine or pyridine.

Examples of suitable groups convertible to Y (or R₁), R₂ and R₅ include those described in the above-mentioned patents or are conventional in the art.

Interconversions of R₈ when hydrogen to C₁₋₆ alkyl may be carried out using conventional alkylation procedures for example using an alkylhalide in the presence of a base.

It should be appreciated that racemates for formula (I) may be resolved or enantiomerically purified compounds of formula (I) may be prepared using procedures conventional in the art and in particular using the procedures outlined in EP-0430631 and EP-0355584.

Suitably, the procedures outlined in or analogous to those described in Example 35 of the present specification may be used to prepare specific enantiomers of any compounds of formulae (I), (Ia), or (Ib).

Compounds of formulae (I), (IIa), (IIb), (IIc) and (IId) may be prepared from readily available starting materials using the procedures outlined or analogous to those described in the above-mentioned patents.

Compounds of formulae (IIIa) and (IIIb) are either commercially available or may be prepared according to conventional procedures known in the art of organic chemistry.

Compounds of formula (I) in which R₅ is hydroxy, R₆ is C₁₋₂ alkyl and R₉ is hydrogen may be prepared according to the procedures outlined in R. Gericke *et al*. J. Med. Chem. Vol.34, p3074(1991).

The following examples and pharmacological test results illustrate the present invention:

The following compounds were prepared by methods analgous to those described in the abovementioned patents publications.

### Example 1

trans-6-Cyano-3,4-dihydro-2,2-dimethyl-4-(3-fluorobenzoylamino)2H-1-benzopyran-3-ol.
Mpt. 193-194°C.

### Example 2

trans-6-Cyano-3,4-dihydro-2,2-dimethyl-4-(2-fluorobenzoylamino)2H-1-benzopyran-3-ol
Mpt.163-5°

### Example 3

trans-6-Trifluoromethoxy-3,4-dihydro-2,2-dimethyl-4-(3-fluorobenzoylamino)2H-1-benzopyran-3-ol
Mpt. 127-30°C

### Example 4

trans-3,4-Dihydro-2,2-dimethyl-4-(4-fluorobenzoylamino)-2H-1-benzopyran-3-ol
Mpt. 174-5°C

### Example 5

trans-6-Cyano-3,4-dihydro-2,2-dimethyl-4-(4-fluorobenzoylamino)2H-1-benzopyran-3-ol,
Mpt. 229-230°C.
3R, 4S isomer (compound B) Mpt. 224-5°C

### Example 6

trans-6-Chloro-3,4-dihydro-2,2-dimethyl-4-(4-fluorobenzoylamino)2H-1-benzopyran-3-ol,
Mpt. 177-9°C

### Example 7

trans-6-Trifluoromethyl-4-(4-fluorobenzoylamino)-3,4-dihydro-2,2-dimethyl-2H-1-benzopyran-3-ol,
Mpt. 185-7°C

### Example 8

trans-6-Cyano-4-(4-fluorobenzoylmethylamino)-3,4-dihydro- 2,2-dimethyl-2H-1-benzopyran-3-ol,
Mpt. 230-4°C

### Example 9

trans-6-Ethylcarbonyl-3,4-dihydro-2,2-dimethyl-4-(4-fluorobenzoylamino)2H-1-benzopyran-3-ol,
Mpt. 205-207°C.

### Example 10

trans-6-Ethylcarbonyl-3,4-dihydro-2,2-dimethyl-4-(4-fluorobenzoylmethylamino)2H-1-benzopyran-3-ol,
Mpt. 210-212°C.

### Example 11

trans-6-Acetyl-4-(4-fluorobenzoylmethylamino)-3,4-dihydro-2,2-dimethyl-2H-1-benzopyran-3-ol,
Mpt. 207-8°C

### Example 12

trans-6-Cyano-3,4-dihydro-4-(4-fluorobenzoylethylamino)-2,2-dimethyl-2H-1-benzopyran-3-ol
mp 172-175 °C

### Example 13

trans-4-(4-Fluorobenzoylamino)-3,4-dihydro-2,2-dimethyl-7-nitro-2H-1-benzopyran-3-ol
mp 231-233 °C

### Example 14

trans-4-(4-Fluorobenzoylamino)-3,4-dihydro-2,2,6-trimethyl-2H-1-benzopyran-3-ol
mp 185-186 °C

### Example 15

trans-6-Ethyl-4-(4-fluorobenzoylamino)-3,4-dihydro-2,2-dimethyl-2H-1-benzopyran-3-ol
mp 235-237 °C

### Example 16

trans-6-Ethyl-4-(4-fluorobenzoylethylamino)-3,4-dihydro-2,2-dimethyl-2H-1-benzopyran-3-of
mp 175 °C

### Example 17

trans-4-(4-Fluorobenzoylamino)-3,4-dihydro-2,2-dimethyl-6-isopropyl-2H-1-benzopyran-3-ol
mp 235-236 °C

### Example 18

trans-6-Acetyl-4-(4-fluorobenzoylamino)-3,4-dihydro-2,2-dimethyl-2H-1-benzopyran-3-ol
mp 203 °C

### Example 19

trans-6-Acetyl-4R-(4-fluorobenzoylamino)-3,4-dihydro-2,2-dimethyl-2H-1-benzopyran-3S-ol
mp 162-163°C

### Example 20

trans-6-Acetyl-4S-(4-fluorobenzoylamino)-3,4-dihydro-2,2-dimethyl-2H-1-benzopyran-3R-ol
mp 164°C

### Example 21

trans-6-Cyano-4S-(3-fluorobenzoylamino)-3,4-dihydro-2,2-dimethyl-2H-1-benzopyran-3R-ol
mp 221°C

### Example 22

trans-6-Cyano-4R-(3-fluorobenzoylamino)-3,4-dihydro-2,2-dimethyl-2H-1-benzopyran-3S-ol
mp 221 °C

### Example 23

trans-6-Ethyl-4-(4-fluorobenzoylmethylamino)-3,4-dihydro-2,2-dimethyl-2H-1-benzopyran-3-ol
mp 162-164 °C

### Example 24

trans-6-Cyano-4S-(2-fluorobenzoylamino)-3,4-dihydro-2,2-dimethyl-2H-1-benzopyran-3R-ol
mp 163-165 °C

### Example 25

trans-6-Cyano-4S-(4-fluorobenzoylamino)-3,4-dihydro-2,2-dimethyl-2H-1-benzopyran-3R-ol
mp 224-225 °C

### Example 26

trans-6-Cyano-4-(4-fluorobenzoylamino)-3,4-dihydro-2,2,3-trimethyl-2H-1-benzopyran-3-ol
mp 218-220 °C

### Example 27

trans-4-(4-Fluorobenzoylamino)-3,4-dihydro-2,2-dimethyl-6-nitro-2H-1-benzopyran-3-ol
mp 197-198 °C

### Example 28

trans-6-Cyano-4S-(2,4-difluorobenzoylamino)-3,4-dihydro-2,2-dimethyl-2H-1-benzopyran-3R-ol
mp 175-176 °C

### Example 29

trans-4-(4-Fluorobenzoylamino)-6-pentafluoroethyl-3,4-dihydro-2,2-dimethyl-2H-1-benzopyran-3-ol
mp 181 °C

### Example 30

trans-4-(4-Fluorobenzoylamino)-3,4-dihydro-6-methoxycarbonyl-2,2-dimethyl-2H-1-benzopyran-3-ol
mp 198 °C

### Example 31

trans-6-Acetyl-4-(3-fluorobenzoylamino)-3,4-dihydro-2,2-dimethyl-2H-1-benzopyran-3-ol
mp 212 °C

### Example 32

trans-4-(4-Fluorobenzoylamino)-3,4-dihydro-2,2-dimethyl-6-phenylsulphonyl-2H-1-benzopyran-3-ol
mp 239-240 °C

### Example 33

trans-4-(4-Fluorobenzoylamino)-3,4-dihydro-2,2-dimethyl-6-phenyl-2H-1-benzopyran-3-ol
mp 164-165 °C

### Example 34

trans-6-Fluoro-4-(4-fluorobenzoylamino)-3,4-dihydro-2,2-dimethyl-2H-1-benzopyran-3-ol
mp 164-165 °C

### Example 35

trans-6-Ethyl-4S-(4-fluorobenzoylamino)-3,4-dihydro-2,2-dimethyl-2H-1-benzopyran-3R-ol

(-)-Mandelic acid was used to resolve the residue from resolution of trans-4-amino-6-ethyl-2,2-dimethyl-chroman-3-ol (to give the 3S, 4R enantiomer - as described in EPA 412 760) under standard resolving conditions using acetone as recrystallisation solvent. This furnished trans-4S-amino-6-ethyl-2,2-dimethylchroman-3R-ol D-mandelate.

The 4S, 3R-aminoalcohol mandelate (4.0 g) was dissolved in dichloromethane (250 ml) and triethylamine (3.34 ml) and cooled in an ice bath. To this stirred solution was added 4-fluorobenzoyl chloride (1.7 g) dropwise. On completion of addition the reaction mixture was allowed to attain room temperature and was stirred overnight. The solvent was evaporated and the residue was taken up in ethyl acetate (150 ml). This solution was washed with 5% sodium bicarbonate solution, and brine, and dried over anhydrous magnesium sulphate. Filtration and evaporation and recrystallisation of the residue from Ethyl acetate-hexane gave the title compound of example 35. mp 132-136°C. αD/20 + 69.1° (methanol, c=1.0).
- NMR(CDCL3)δ: 1.21 (3H, t, J = 8 Hz) 1.28 (3H, s) 1.49 (3H, s) 2.88 (2H, q, J = 8 Hz) 3.76 (1H, d, J = 9 Hz) 4.46 (1H, broad s) 5.21 (1H, t, J = 9,8 Hz) 6.41 (1H, d, J = 8 Hz) 6.80 (1H, d, J = 9 Hz) 7.07 (2H, irregular m) 7.17 (2H, t, J = 9 Hz) 7.74 (2H, m)

### Example 36

trans-4R-(4-Fluorobenzoylamino)-3,4-dihydro-6-(1-hydroxyethyl)-2,2-dimethyl-2H-1-benzopyran-3S-ol
mp 132-133 °C

### Example 37

trans-4S-(4-Fluorobenzoylamino)-3,4-dihydro-6-(1-hydroxyethyl)-2,2-dimethyl-2H-1-benzopyran-3R-ol
mp 133-134 °C

### Example 38

trans-4-(2-Fluorobenzoylamino)-3,4-dihydro-2,2-dimethyl-2H-pyrano[3,2-c]pyridin-3-ol
mp 254 °C

### Example 39

trans-4-(3-Fluorobenzoylamino)-3,4-dihydro-2,2-dimethyl-2H-pyrano[3,2-c]pyridin-3-ol
mp 259-261 °C

### Example 40

trans-4-(4-Fluorobenzoylamino)-3,4-dihydro-2,2-dimethyl-2H-pyrano[3,2-c]pyridin-3-ol
mp 254-255 °C

### Example 41

trans-6-Cyano-4-(3,4-difluorbenzoyl-methylamino)-3,4-dihydro-2,2-dimethyl-2H-1-benzopyran-3-ol
mp 199-200°C

### Example 42

trans-4-(4-fluorobenzoyl-methylamino)-6-trifluoromethyl-3,4-dihydro-2,2-dimethyl-2H-1-benzopyran-3-ol
mp 196-197°C

### Example 43

trans-4-(4-fluorobenzoyl-methylamino)-3,4-dihydro-2,2-dimethyl-2H-1-benzopyran-3-ol
mp 160-161°C

### Example 44

trans-6-cyano-4S-(3,4-difluorobenzoylamino)-3,4-dihydro-2,2-dimethyl-2H-1-benzopyran-3R-ol
mp 227°C

### Example 45

trans-6-Cyano-4-(4-fluorobenzoylamino)-2,2-dimethyl-1,2,3,4-tetrahydroquinolin-3-ol
mp 244-248 °C

### Example 46

trans-6-t-Butyl-4-(4-fluorobenzoylamino)-3,4-dihydro-2,2-dimethyl-2H-1-benzopyran-3-ol
mp 163-166 °C

### Example 47

trans-4-(4-Fluorobenzoylamino)-3,4-dihydro-6-iodo-2,2-dimethyl-2H-1-benzopyran-3-ol
mp 204-205 °C

### Example 48

trans-4-(4-Fluorobenzoylamino)-3,4-dihydro-2,2-dimethyl-6-cyclopentyl-2H-1-benzopyran-3-ol
mp 173-174 °C

### Example 49

trans- 6-Aminocarbonylmethyl-4-(4-fluorobenzoylamino)-3,4-dihydro-2,2-dimethyl-2H-1-benzopyran-3-ol
mp 190 °C

### Example 50

trans- 4-(4-Fluorobenzoylamino)-3,4-dihydro-6-methoxy-2,2-dimethyl-2H-1-benzopyran-3-ol
mp 155-156 °C

### PHARMACOLOGICAL DATA

### 1. Geller-Seifter Procedure

Potential anxiolytic properties are evaluated using the Geller-Seifter procedure based on that originally described by Geller and Seifter, (1960) Psychopharmacologia, 1, 482-492. This procedure has been shown to be selective for drugs with anxiolytic properties (Cook and Sepinwall, (1975) "Mechanism of Action of Benzodiazepines" ed. Costa, E. and Greengard, P., Raven Press, New York, pp. 1-28).

Rats are trained on a variable interval 30 sec schedule (VI30) to press a lever in order to obtain food reward. The 5 min sessions of the VI30 schedule alternate with 2-5 min of a schedule (FR5) in which every 5th lever press is followed by presentation of a food pellet paired with a 0.5 sec mild footshock. The total study lasts approximately 30 mins. Rats typically respond with high rates of lever pressing under the VI30 schedule and low response rates under the FR5 'conflict' session. Anxiolytic drugs increase the suppressed response rates of rats in a 'conflict' session.

Drugs are administered intraperitoneally or orally to groups of 3-8 rats 30 to 60 mins before testing.

The results are expressed as the percentage increase in the square root of the total number of lever presses in the FR5 'conflict' session. Square root transformation is necessary to normalise the data for statistical analysis using parametric methods.

The compound of Example 4 showed a significant increase in responding in the 'conflict' session at a dose of 10mg/kg p.o.

### 2. MES TEST

The maximal electroshock seizure (MES) test in rodents is one of the most widely used models of human grand mal epilepsy¹. In this model, anticonvulsant agents elevate the threshold to electrically-induced seizures whilst proconvulsants lower the seizure threshold.

### Method

Mice (male, Charles River, U.K CD-1 strain, 25-30g) are randomly assigned to groups of 10-20 and dosed orally or intraperitoneally at a dose volume of 10ml/kg with various doses of compound (1-100 mg/kg) or vehicle. Mice are then subjected at 30 or 60 min post dose to a variable voltage electroshock (0.1 sec., 50 Hz, sine wave form) via a buccal and a subcutaneous electrode. The mean voltage and standard error required to induce a tonic seizure in 50% (CV₅₀) of the mice in the group is determined by the 'up and down' method of Dixon and Mood (1948)². Statistical comparisons between vehicle- and drug-treated groups are made using the method of Litchfield and Wilcoxon (1949)³.

In control animals the CV₅₀ is usually 40 - 50V. Hence the first animal in the control group is subjected to a voltage of 45V. If a tonic seizure does not ensue, the voltage is increased for a subsequent mouse. If a tonic convulsion does occur, then the voltage is decreased, and so on until all the animals in the group have been tested.

The percentage increase or decrease in CV₅₀ for each group compared to the control is calculated.

Studies are carried out using a Heathkit shock generator with totally variable control of shock level from 0 to 200V and voltage steps of 5V are used.

Drugs are suspended in 1% methyl cellulose.

### Reference

1. Swinyard, E.A. (1972). Electrically-induced convulsions. In: Experimental Models of Epilepsy ed. Purpura, D.P. et al., 433-458, Raven Press, New York.
2. Dixon, W.J. and Mood, A.M. (1948). J. Amer. Stat. Assn., 43, 109-126.
3. Litchfield, J.T. and Wilcoxon, F. (1949). J. Pharmacol. Exp. Ther. 96, 99-113.

### Results

Compounds of Examples 1-3, 5, 7, 8, 18, 20, 21, 25, 30, 31, 35 and 37 and compound X showed a significant increase in CV₅₀ at a dose of 10 mg/kg p.o.

### 3. X-Maze

### Introduction

The X-maze test of anxiety (Handley and Mithani, 1984) examines the exploratory response of naive rats in an environment which offers both anxiogenic (open arms) and relatively non-anxiogenic (closed arms) areas. A selective increase in exploration of the open arms following drug pretreatment is therefore postulated to indicate anxiolytic effects.

### Method

The X-maze was raised 70cm above the floor and consisted of two enclosed arms 45cm (long) x 15cm (wide) x 10cm (high) and two open arms 45 x 10 x 1cm arranged such that the two arms of each type were opposite each other. Both arm types were marked into two equal sections. Rats were placed onto the centre of the X-maze and observed for a period of 10 minutes during which time the following parameters were recorded: 1) the number of entries onto, and the time spent on, (a) open arms, (b) closed arms, (c) end of open arms and (d) end of closed arms. 2) the number of sections crossed. The fear-drive evoked in the open arms exceeds that in the enclosed arms and rats typically show a clear preference for the enclosed arms. Anxiolytic drugs increase the number of entries made onto, and the time spent on, the outer half of the open arms, and also the percentage of entries made onto, and the time spent on, the whole of the open arms. These four measures of anxiety, and also the total number of sections traversed, were calculated for each animal. Drugs are administered intraperitoneally or orally to groups of 6 to 12 rats 30 to 60 mins before testing. Statistical comparisons between vehicle- and drug-treated groups were made using a Mann-Whitney 'U' test (two tailed).
S.L. Handley and S. Mithani, Arch. Pharmacol., 1984 327 1-5

### RESULTS

The compound of Example 21, caused a significant increase in open arm entries at a dose of 30 mg/kg p.o.

## Claims

1. The use of a compound of formula (I), or a pharmaceutically acceptable salt thereof; 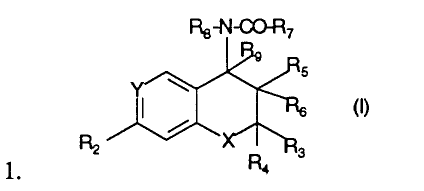 wherein:
either Y is N and R₂ is hydrogen, or Y is C-R₁
where:
either one of R₁ and R₂ is hydrogen and the other is selected from hydrogen, C₃₋₈ cycloalkyl, C₁₋₆ alkyl optionally interupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is-CF₂-, -CO-, -CH₂- or CH(OH), trifluoromethoxy, C₁₋₆ alkylsulphinyl, C₁₋₆ alkylsulphonyl, C₁₋₆ alkoxysulphinyl, C₁₋₆ alkoxysulphonyl, aryl, heteroaryl, arylcarbonyl, heteroarylcarbonyl, arylsulphinyl, heteroarylsulphinyl, arylsulphonyl, heteroarylsulphonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, any amino moiety being optionally substituted by one or two C₁₋₆ alkyl groups, or C₁₋₆ alkylsulphinylamino, C₁₋₆ alkylsulphonylamino,C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl;
one of R₃ and R₄ is hydrogen or C₁₋₄ alkyl and the other is C₁₋₄ alkyl or R₃ and R₄ together are C₂₋₅ polymethylene;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ is hydrogen or C₁₋₂ alkyl and R₉ is hydrogen;
R₇ is fluorophenyl;
R₈ is hydrogen or C₁₋₆ alkyl;
the R₈-N-CO-R₇ group being trans to the R₅ group;
and X is oxygen or NR₁₀ where R₁₀ is hydrogen or C₁₋₆ alkyl;
wherein aryl means phenyl or naphthyl and heteroaryl means a 5- or 6-membered monocyclic or 9- or 10-membered bicyclic group containing up to three heteroatoms which are the same or different and are selected from oxygen, nitrogen and sulphur; and each aryl or heteroaryl group is optionally substituted with up to three substituents independently selected from C₁₋₄alkyl, C₁₋₄alkoxy, halo, hydroxy, nitro, cyano and SOₙH where n=0 to 2;
for the manufacture of a medicament for the treatment and/or prophylaxis of disorders treatable or preventable with anti-convulsive agents.

2. A use according to claim 1 in which the compound of formula (I) has the following variables wherein;
Y is C-R₁, where:
either one of R₁ and R₂ is hydrogen and the other is selected from hydrogen, C₁₋₆ alkylcarbonyl, C₁₋₆ alkoxycarbonyl, C₁₋₆ alkylcarbonyloxy, C₁₋₆ alkylhydroxymethyl, nitro, cyano, chloro, trifluoromethyl, trifluoromethoxy, C₁₋₆ alkylsulphinyl, C₁₋₆ alkylsulphonyl, C₁₋₆ alkoxysulphinyl, C₁₋₆ alkoxysulphonyl, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, the amino moiety being optionally substituted by one or two C₁₋₆ alkyl groups, or C₁₋₆ alkylsulphinylamino, C₁₋₆ alkylsulphonylamino, C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl;
one of R₃ and R₄ is hydrogen or C₁₋₄ alkyl and the other is C₁₋₄ alkyl or R₃ and R₄ together are C₂₋₅ polymethylene;
R₅ is hydroxy and R₆ is hydrogen;
R₇ is fluorophenyl;
R₈ is hydrogen or C₁₋₆ alkyl; and
R₉ is hydrogen;
the R₈-N-CO-R₇ group being trans to the R₅ group.

3. Use according to claim 1 wherein the compound is selected from :
trans-6-Cyano-3,4-dihydro-2,2-dimethyl-4-(3-fluorobenzoylamino)2H-1-benzopyran-3-ol,
trans-6-Cyano-3,4-dihydro-2,2-dimethyl-4-(2-fluorobenzoylamino)2H-1-benzopyran-3-ol,
trans-6-Trifluoromethoxy-3,4-dihydro-2,2-dimethyl-4-(3-fluorobenzoylamino)2H-1-benzopyran-3-ol,
trans-6-Cyano-4S-(3-fluorobenzoylamino)-3,4-dihydro-2,2-dimethyl-2H-1-benzopyran-3R-ol,
trans-6-Cyano-4R-(3-fluorobenzoylamino)-3,4-dihydro-2,2-dimethyl-2H-1-benzopyran-3S-ol,
trans-6-Cyano-4S-(2-fluorobenzoylamino)-3,4-dihydro-2,2-dimethyl-2H-1-benzopyran-3R-ol,
trans-6-Cyano-4S-(2,4-difluorobenzoylamino)-3,4-dihydro-2,2-dimethyl-2H-1-benzopyran-3R-ol,
trans-6-Acetyl-4-(3-fluorobenzoylamino)-3,4-dihydro-2,2-dimethyl-2H-1-benzopyran-3-ol,
trans-6-Cyano-4-(3,4-difluorobenzoyl-methylamino)-3,4-dihydro-2,2-dimethyl-2H-1-benzopyran-3-ol and
trans-6-Cyano-4S-(3,4-difluorobenzoylamino)-3,4-dihydro-2,2-dimethyl-2H-1-benzopyran-3R-ol or a pharmaceutically acceptable salt thereof.

4. Use according to claim 1 wherein the compound of formula (I) is a compound of formula (Ib) or pharmaceutically acceptable salts thereof: 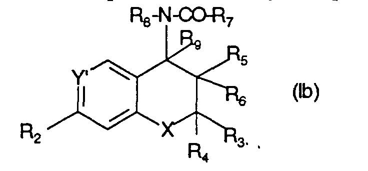 wherein variables R₃, R₄, R₅, R₆, R₇, R₈, R₉ and X are as defined in relation to formula (I) in claim 1 and Y' is CR₁ where R₁ and R₂ are both hydrogen or one of R₁ and R₂ is trifluoromethoxy, C₁₋₆ alkyl interrupted with oxygen or substituted with hydroxy, C₁₋₆ alkoxy or substituted amino-carbonyl, CF₃A-(where A is -CF₂-, -CO-, -CH₂ or CH(OH) ), aryl sulphonyl, aryl, C₃₋₈ cycloalkyl, C₁₋₆ alkoxy, heteroaryl, arylcarbonyl, heteroarylcarbonyl, arylsulphinyl, heteroarylsulphinyl, heteroarylsulphonyl, in which any aromatic moiety is optionally substituted, and the other is hydrogen.

5. Use according to claim 4 wherein the compound is selected from :
trans-3,4-Dihydro-2,2-dimethyl-4-(4-fluorobenzoylamino)-2H-1-benzopyran-3-ol,
trans-4-(4-Fluorobenzoylamino)-3,4-dihydro-2,2,6-trimethyl-2H-1-benzopyran-3-ol,
trans-6-Ethyl-4-(4-fluorobenzoylamino)-3,4-dihydro-2,2-dimethyl-2H-1-benzopyran-3-ol,
trans-6-Ethyl-4-(4-fluorobenzoylethylamino)-3,4-dihydro-2,2-dimethyl-2H-1-benzopyran-3-ol,
trans-6-Ethyl-4-(4-fluorobenzoylmethylamino)-3,4-dihydro-2,2-dimethyl-2H-1-benzopyran-3-ol,
trans-4-(4-Fluorobenzoylamino)-6-pentafluoroethyl-3,4-dihydro-2,2-dimethyl-2H-1-benzopyran-3-ol,
trans-4-(4-Fluorobenzoylamino)-3,4-dihydro-2,2-dimethyl-6-phenylsulphonyl-2H-1-benzopyran-3-ol,
trans-4-(4-Fluorobenzoylamino)-3,4-dihydro-2,2-dimethyl-6-phenyl-2H-1-benzopyran-3-ol,
trans-6-Ethyl-4S-(4-fluorobenzoylamino)-3,4-dihydro-2,2-dimethyl-2H-1-benzopyran-3R-ol,
trans-4R-(4-Fluorobenzoylamino)-3,4-dihydro-6-(1-hydroxyethyl)-2,2-dimethyl-2H-1-benzopyran-3S-ol,
trans-4S-(4-Fluorobenzoylamino)-3,4-dihydro-6-(1-hydroxyethyl)-2,2-dimethyl-2H-1-benzopyran-3R-ol,
trans-4-(4-Fluorobenzoyl-methylamino)-3,4-dihydro-2,2-dimethyl-2H-1-benzopyran-3-ol,
trans-6-t-Butyl-4-(4-fluorobenzoylamino)-3,4-dihydro-2,2-dimethyl-2H-1-benzopyran-3-ol,
trans-4-(4-Fluorobenzoylamino)-3,4-dihydro-2,2-dimethyl-6-cyclopentyl-2H-1-benzopyran-3-ol and
trans- 4-(4-Fluorobenzoylamino)-3,4-dihydro-6-methoxy-2,2-dimethyl-2H-1-benzopyran-3-ol, or a pharmaceutically acceptable salt thereof.

6. Use according to claim 1 wherein the compound is selected from :
trans-6-Cyano-3,4-dihydro-2,2-dimethyl-4-(4-fluorobenzoylamino)2H-1-benzopyran-3-ol,
trans-6-Chloro-3,4-dihydro-2,2-dimethyl-4-(4-fluoro-benzoylamino)2H-1-benzopyran-3-ol,
trans-6-Trifluoromethyl-4-(4-fluorobenzoylamino)-3,4-dihydro-2,2-dimethyl-2H-1-benzopyran-3-ol,
trans-6-Cyano-4-(4-fluorobenzoylmethylamino)-3,4-dihydro- 2,2-dimethyl-2H-1-benzopyran-3-ol,
trans-6-Ethylcarbonyl-3,4-dihydro-2,2-dimethyl-4-(4-fluorobenzoylamino)2H-1-benzopyran-3-ol,
trans-6-Ethylcarbonyl-3,4-dihydro-2,2-dimethyl-4-(4-fluorobenzoylmethylamino)2H-1-benzopyran-3-ol,
trans-6-Acetyl-4-(4-fluorobenzoylmethylamino)-3,4-dihydro-2,2-dimethyl-2H-1-benzopyran-3-ol,
trans-6-Cyano-3,4-dihydro-4-(4-fluorobenzoylethylamino)-2,2-dimethyl-2H-1-benzopyran-3-ol,
trans-4-(4-Fluorobenzoylamino)-3,4-dihydro-2,2-dimethyl-7-nitro-2H-1-benzopyran-3-ol,
trans-6-Acetyl-4R-(4-fluorobenzoylamino)-3,4-dihydro-2,2-dimethyl-2H-1-benzopyran-3S-ol,
trans-6-Acetyl-4S-(4-fluorobenzoylamino)-3,4-dihydro-2,2-dimethyl-2H-1-benzopyran-3R-ol,
trans-6-Cyano-4S-(4-fluorobenzoylamino)-3,4-dihydro-2,2-dimethyl-2H-1-benzopyran-3R-ol,
trans-6-Cyano-4-(4-fluorobenzoylamino)-3,4-dihydro-2,2,3-trimethyl-2H-1-benzopyran-3-ol,
trans-4-(4-Fluorobenzoylamino)-3,4-dihydro-2,2-dimethyl-6-nitro-2H-1-benzopyran-3-ol,
trans-4-(4-Fluorobenzoylamino)-3,4-dihydro-6-methoxycarbonyl-2,2-dimethyl-2H-1-benzopyran-3-ol,
trans-6-Fluoro-4-(4-fluorobenzoylamino)-3,4-dihydro-2,2-dimethyl-2H-1-benzopyran-3-ol,
trans-4-(4-Fluorobenzoylmethylamino)-6-trifluoromethyl-3,4-dihydro-2,2-dimethyl-2H-1-benzopyran-3-ol,
trans-4-(4-Fluorobenzoylamino)-3,4-dihydro-6-iodo-2,2-dimethyl-2H-1-benzopyran-3-ol and
trans- 6-Aminocarbonylmethyl-4-(4-fluorobenzoylamino)-3,4-dihydro-2,2-dimethyl-2H-1-benzopyran-3-ol or a pharmaceutically acceptable salt thereof.

7. Use according to claim 4 wherein R₃ and R₄ are both methyl.

8. Use according to claim 4 wherein R₅ is hydroxy and R₆ and R₉ are hydrogen.

9. Use according to claim 4 wherein X is oxygen.

10. Use according to claim 4 wherein Y is CR₁ and R₁ is cyano, methoxy, trifluoromethoxy, chloro, trifluoromethyl, ethylcarbonyl, acetyl, hydrogen, methyl, ethyl, iso-propyl,tertiary-butyl, nitro, C₂F₅, methoxycarbonyl, phenylsulphonyl, phenyl, fluoro, iodo, cyclopentyl, aminocarbonylmethyl and 1-hydroxyethyl and R₂ is hydrogen

11. Use according to claim 4 wherein R₁ is cyano, ethyl or acetyl and R₂ is hydrogen.

12. Use according to claim 4 wherein R₇ is 2-fluorophenyl, 3-fluorophenyl, 4-fluorophenyl, 2,4-difluorophenyl or 3,4-difluorophenyl.

13. Use according to claim 4 wherein R₈ is hydrogen, methyl or ethyl.

14. Use according to claim 1 or 4 wherein the compound has the 4S,3R configuration.

15. Use according to claim 14 wherein the compound is trans-6-Acetyl-4S-(4-fluorobenzoylamino)-3,4-dihydro-2,2-dimethyl-2H-1-benzopyran-3R-ol,

## Patentansprüche

1. Verwendung einer Verbindung der Formel (I) oder eines pharmazeutisch verträglichen Salzes davon 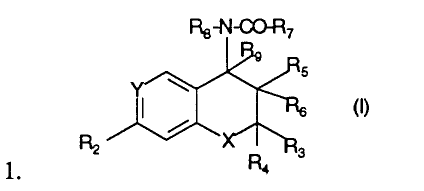 in der entweder Y N bedeutet und R₂ ein Wasserstoffatom bedeutet oder Y für C-R₁ steht, wobei
einer der Reste R₁ und R₂ ein Wasserstoffatom bedeutet und der andere aus einem Wasserstoffatom, einem C₃₋₈-Cycloalkylrest, einem C₁₋₆-Alkylrest, gegebenenfalls durch ein Sauerstoffatom unterbrochen oder durch eine Hydroxygruppe substituiert, einem C₁₋₆-Alkoxyrest oder einem substituierten Aminocarbonyl-, C₁₋₆-Alkylcarbonyl-, C₁₋₆-Alkoxycarbonyl-, C₁₋₆-Alkylcarbonyloxy-, C₁₋₆-Alkoxyrest, einer Nitro-, Cyanogruppe, einem Halogenatom, einer Trifluormethylgruppe, einem Rest CF₃S oder CF₃-A-, wobei A für -CF₂-, -CO-, -CH₂- oder CH(OH) steht, einer Trifluormethoxygruppe, einem C₁₋₆-Alkylsulfinyl-, C₁₋₆-Alkylsulfonyl-, C₁₋₆-Alkoxysulfinyl-, C₁₋₆-Alkoxysulfonyl-, Aryl-, Heteroaryl-, Arylcarbonyl-, Heteroarylcarbonyl-, Arylsulfinyl-, Heteroarylsulfinyl-, Arylsulfonyl-, Heteroarylsulfonylrest, bei denen jede aromatische Einheit gegebenenfalls substituiert ist, einem C₁₋₆-Alkylcarbonylamino-, C₁₋₆-Alkoxycarbonylamino-, C₁₋₆-Alkylthiocarbonyl-, C₁₋₆-Alkoxythiocarbonyl-, C₁₋₆-Alkylthiocarbonyloxy-, 1-Mercapto-C₂₋₇-alkylrest, einer Formylgruppe oder einem Aminosulfinyl-, Aminosulfonyl- oder Aminocarbonylrest, wobei jede Amino-Einheit gegebenenfalls durch einen oder zwei C₁₋₆-Alkylreste substituiert ist, oder einem C₁₋₆, Alkylsulfinylamino-, C₁₋₆-Alkylsulfonylamino-, C₁₋₆-Alkoxysulfinylamino- oder C₁₋₆-Alkoxysulfonylaminorest oder einem Ethylenylrest, endständig substituiert durch einen C₁₋₆-Alkylcarbonylrest, eine Nitrogruppe oder Cyanogruppe, oder einem Rest -C(C₁₋₆-Alkyl)NOH oder -C(C₁₋₆-Alkyl)NNH₂ ausgewählt ist oder einer der Reste R₁ und R₂ eine Nitrogruppe, Cyanogruppe oder einen C₁₋₃-Alkylcarbonylrest bedeutet und der andere eine Methoxy- oder Aminogruppe, gegebenenfalls durch einen oder zwei C₁₋₆-Alkylreste oder durch einen C₂₋₇-Alkanoylrest substituiert, bedeutet;
einer der Reste R₃ und R₄ ein Wasserstoffatom oder einen C₁₋₄-Alkylrest bedeutet und der andere einen C₁₋₄-Alkylrest bedeutet oder R₃ und R₄ zusammen einen C₂₋₅-Polymethylenrest bedeuten;
R₅ einen C₁₋₆-Alkylcarbonyloxyrest, eine Benzoyloxygruppe, einen Rest ONO₂, cine Benzyloxygruppe, Phenyloxygruppe oder einen C₁₋₆-Alkoxyrest bedeutet und R₆ und
R₉ ein Wasserstoffatom bedeuten oder R₅ eine Hydroxygruppe bedeutet und R₆ ein Wasserstoffatom oder einen C₁₋₂-Alkylrest bedeutet und R₉ ein Wasserstoffatom bedeutet;
R₇ eine Fluorphenylgruppe bedeutet;
R₈ ein Wasserstoffatom oder einen C₁₋₆-Alkylrest bedeutet;
wobei der Rest R₈-N-CO-R₇ in trans-Stellung zu dem Rest R₅ steht;
und X ein Sauerstoffatom oder einen Rest NR₁₀ bedeutet, wobei R₁₀ ein Wasserstoffatom oder einen C₁₋₆-Alkylrest bedeutet;
wobei der Arylrest eine Phenyl- oder Naphthylgruppe bedeutet und der Heteroarylrest einen 5- oder 6-gliedrigen monocyclischer oder 9- oder 10-gliedrigen bicyclischen Rest bedeutet, der bis zu 3 Heteroatome enthält, die gleich oder verschieden sind und aus einem Sauerstoff-, Stickstoff- oder Schwefelatom ausgewählt sind; und jeder Aryl- oder Heteroarylrest gegebenenfalls mit bis zu 3 Substituenten substituiert ist, die unabhängig aus einem C₁₋₄-Alkylrest, C₁₋₄-Alkoxyrest, einem Halogenatom, einer Hydroxy-, Nitro-, Cyanogruppe und einem Rest SOₙH ausgewählt sind, wobei n=0 bis 2;
zur Herstellung eines Medikaments zur Behandlung und/oder Prophylaxe von Störungen, die mit Antikonvulsiva behandel- oder vermeidbar sind.

2. Verwendung nach Anspruch 1, bei welcher die Verbindung der Formel (I) die folgenden Variablen aufweist, wobei:
Y für C-R₁ steht, wobei
einer der Reste R₁ und R₂ ein Wasserstoffatom bedeutet und der andere aus einem Wasserstoffatom, einem C₁₋₆-Alkylcarbonyl-, C₁₋₆-Alkoxycarbonyl-, C₁₋₆-Alkylcarbonyloxy-, C₁₋₆-Alkylhydroxymethylrest, einer Nitro-, Cyanogruppe, einem Chloratom, einer Trifluormethylgruppe, Trifluormethoxygruppe, einem C₁₋₆-Alkylsulfinyl-, C₁₋₆-Alkylsulfonyl-, C₁₋₆-Alkoxysulfinyl-, C₁₋₆-Alkoxysulfonyl-, C₁₋₆-Alkylcarbonylamino-, C₁₋₆-Alkoxycarbonylamino-, C₁₋₆-Alkylthiocarbonyl-, C₁₋₆-Alkoxythiocarbonyl-, C₁₋₆-Alkylthiocarbonyloxy-, 1-Mercapto-C₂₋₇-alkylrest, einer Formylgruppe oder einem Aminosulfinyl-, Aminosulfonyl- oder Aminocarbonylrest, wobei die Amino-Einheit gegebenenfalls durch ein oder zwei C₁₋₆-Alkylreste substituiert ist, oder einem C₁₋₆-Alkylsulfinylamino-, C₁₋₆-Alkylsulfonylamino-, C₁₋₆-Alkoxysulfinylamino- oder C₁₋₆-Alkoxysulfonylamino oder einem Ethylenylrest, endständig substituiert durch einen C₁₋₆-Alkylcarbonylrest, eine Nitro- oder Cyanogruppe, oder einem Rest -C(C₁₋₆-Alkyl)NOH oder -C(C₁₋₆-Alkyl)NNH₂ ausgewählt ist oder einer der Reste R₁ und R₂ eine Nitro-, Cyanogruppe oder einen C₁₋₃-Alkoxycarbonylrest bedeutet und der andere eine Methoxy- oder Aminogruppe, gegebenenfalls substituiert durch einen oder zwei C₁₋₆-Alkylreste oder durch einen C₂₋₇-Alkanoylrest, bedeutet;
einer der Reste R₃ und R₄ ein Wasserstoffatom oder einen C₁₋₄-Alkylrest bedeutet und der andere einen C₁₋₄-Alkylrest bedeutet oder R₃ und R₄ zusammen einen C₂₋₅-Polymethylenrest bedeuten;
R₅ eine Hydroxygruppe bedeutet und R₆ ein Wasserstoffatom bedeutet;
R₇ eine Fluorphenylgruppe bedeutet;
R₈ ein Wasserstoffatom oder einen C₁₋₆-Alkylrest bedeutet; und
R₉ ein Wasserstoffatom bedeutet;
wobei der Rest R₈-N-CO-R₇ in trans-Stellung zu dem Rest R₅ steht.

3. Verwendung nach Anspruch 1, wobei die Verbindung aus:
trans-6-Cyano-3,4-dihydro-2,2-dimethyl-4-(3-fluorbenzoylamino)2H-1-benzopyran-3-ol,
trans-6-Cyano-3,4-dihydro-2,2-dimethyl-4-(2-fluorbenzoylamino)2H-1-benzopyran-3-ol,
trans-6-Trifluormethoxy-3,4-dihydro-2,2-dimethyl-4-(3-fluorbenzoylamino)2H-1-benzopyran-3-ol,
trans-6-Cyano-4S-(3-fluorbenzoylamino)-3,4-dihydro-2,2-dimethyl-2H-1-benzopyran-3R-ol,
trans-6-Cyano-4R-(3-fluorbenzoylamino)-3,4-dihydro-2,2-dimethyl-2H-1-benzopyran-3S-ol.
trans-6-Cyano-4S-(2-fluorbenzoylamino)-3,4-dihydro-2,2-dimethyl-2H-1-benzopyran-3R-ol,
trans-6-Cyano-4S-(2,4-difluorbenzoylamino)-3,4-dihydro-2,2-dimethyl-2H-1-benzopyran-3R-ol,
trans-6-Acetyl-4-(3-fluorbenzoylamino)-3,4-dihydro-2,2-dimethyl-2H-1-benzopyran-3-ol,
trans-6-Cyano-4-(3,4-difluorbenzoylmethylamino)-3,4-dihydro-2,2-dimethyl-2H-1-benzopyran-3-ol und
trans-6-Cyano-4S-(3,4-difluorbenzoylamino)-3,4-dihydro-2,2-dimethyl-2H-1-benzopyran-3R-ol oder einem pharmazeutisch verträglichen Salz davon ausgewählt ist.

4. Verwendung nach Anspruch 1, wobei die Verbindung der Formel (I) eine Verbindung der Formel (Ib) oder pharmazeutisch verträgliche. Salze davon darstellt: 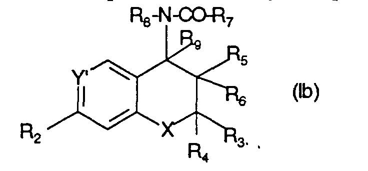 wobei die Variablen R₃, R₄, R₅, R₆, R₇, R₈, R₉ und X wie mit Bezug auf Formel (I) in Anspruch 1 definiert sind und Y' CR₁ bedeutet, wobei R₁ und R₂ beide ein Wasserstoffatom bedeuten oder einer der Reste R₁ und R₂ eine Trifluormethoxygruppe, einen C₁₋₆-Alkylrest, durch ein Sauerstoffatom unterbrochen oder durch eine Hydroxygruppe substituiert, einen C₁₋₆-Alkoxyrest oder einen substituierten Aminocarbonylrest, einen Rest CF₃A- (wobei A für -CF₂-, -CO-, -CH₂ oder CH(OH) steht), einen Arylsulfonyl-, Aryl-, C₃₋₈-Cycloalkyl-, C₁₋₆-Alkoxy-, Heteroaryl-, Arylcarbonyl-, Heteroarylcarbonyl-, Arylsulfinyl-, Heteroarylsulfinyl-, Heteroarylsulfonylrest bedeutet, in denen jede aromatische Einheit gegebenenfalls substituiert ist, und der andere ein Wasserstoffatom bedeutet.

5. Verwendung nach Anspruch 4, wobei die Verbindung aus:
trans-3,4-Dihydro-2,2-dimethyl-4-(4-fluorbenzoylamino)-2H-1-benzopyran-3-ol,
trans-4-(4-Fluorbenzoylamino)-3,4-dihydro-2,2,6-trimethyl-2H-1-benzopyran-3-ol,
trans-6-Ethyl-4-(4-fluorbenzoylamino)-3,4-dihydro-2,2-dimethyl-2H-1-benzopyran-3-ol,
trans-6-Ethyl-4-(4-fluorbenzoylethylamino)-3,4-dihydro-2,2-dimethyl-2H-1-benzopyran-3-ol,
trans-6-Ethyl-4-(4-fluorbenzoylmethylamino)-3,4-dihydro-2,2-dimethyl-2H-1-benzopyran-3-ol,
trans-4-(4-Fluorbenzoylamino)-6-pentafluorethyl-3,4-dihydro-2,2-dimethyl-2H-1-benzopyran-3-ol,
trans-4-(4-Fluorbenzoylamino)-3,4-dihydro-2,2-dimethyl-6-phenylsulfonyl-2H-1-benzopyran-3-ol,
trans-4-(4-Fluorbenzoylamino)-3,4-dihydro-2,2-dimethyl-6-phenyl-2H-1-benzopyran-3-ol,
trans-6-Ethyl-4S-(4-fluorbenzoylamino)-3,4-dihydro-2,2-dimethyl-2H-1-benzopyran-3R-ol,
trans-4R-(4-Fluorbenzoylamino)-3,4-dihydro-6-(1-hydroxyethyl)-2,2-dimethyl-2H-1-benzopyran-3S-ol,
trans-4S-(4-Fluorbenzoylamino)-3,4-dihydro-6-(1-hydroxyethyl)-2,2-dimethyl-2H-1-benzopyran-3R-ol,
trans-4-(4-Fluorbenzoylmethylamino)-3,4-dihydro-2,2-dimethyl-2H-1-benzopyran-3-ol,
trans-6-t-Butyl-4-(4-fluorbenzoylamino)-3,4-dihydro-2,2-dimethyl-2H-1-benzopyran-3-ol,
trans-4-(4-Fluorbenzoylamino)-3,4-dihydro-2,2-dimethyl-6-cyclopentyl-2H-1-benzopyran-3-ol und
trans-4-(4-Fluorbenzoylamino)-3,4-dihydro-6-methoxy-2,2-dimethyl-2H-1-benzopyran-3-ol oder einem pharmazeutisch verträglichen Salz davon ausgewählt ist.

6. Verwendung nach Anspruch 1, wobei die Verbindung aus:
trans-6-Cyano-3,4-dihydro-2,2-dimethyl-4-(4-fluorbenzoylamino)2H-1-benzopyran-3-ol,
trans-6-Chlor-3,4-dihydro-2,2-dimethyl-4-(4-fluorbenzoylamino)2H-1-benzopyran-3-ol,
trans-6-Trifluormethyl-4-(4-fluorbenzoylamino)-3,4-dihydro-2,2-dimethyl-2H-1-benzopyran-3-ol,
trans-6-Cyano-4-(4-fluorbenzoylmethylamino)-3,4-dihydro-2,2-dimethyl-2H-1-benzopyran-3-ol,
trans-6-Ethylcarbonyl-3,4-dihydro-2,2-dimethyl-4-(4-fluorbenzoylamino)2H-1-benzopyran-3-ol,
trans-6-Ethylcarbonyl-3,4-dihydro-2,2-dimethyl-4-(4-fluorbenzoylmethylamino)2H-1-benzopyran-3-ol,
trans-6-Acetyl-4-(4-fluorbenzoylmethylamino)-3,4-dihydro-2,2-dimethyl-2H-1-benzopyran-3-ol,
trans-6-Cyano-3,4-dihydro-4-(4-fluorbenzoylethylamino)-2,2-dimethyl-2H-1-benzopyran-3-ol,
trans-4-(4-Fluorbenzoylamino)-3,4-dihydro-2,2-dimethyl-7-nitro-2H-1-benzopyran-3-ol,
trans-6-Acetyl-4R-(4-fluorbenzoylamino)-3,4-dihydro-2,2-dimethyl-2H-1-benzopyran-3S-ol,
trans-6-Acetyl-4S-(4-fluorbenzoylamino)-3,4-dihydro-2,2-dimethyl-2H-1-benzopyran-3R-ol,
trans-6-Cyano-4S-(4-fluorbenzoylamino)-3,4-dihydro-2,2-dimethyl-2H-1-benzopyran-3R-ol,
trans-6-Cyano-4-(4-fluorbenzoylamino)-3,4-dihydro-2,2,3-trimethyl-2H-1-benzopyran-3-ol,
trans-4-(4-Fluorbenzoylamino)-3,4-dihydro-2,2-dimethyl-6-nitro-2H-1-benzopyran-3-ol,
trans-4-(4-Fluorbenzoylamino)-3,4-dihydro-6-methoxycarbonyl-2,2-dimethyl-2H-1-benzopyran-3-ol,
trans-6-Fluor-4-(4-fluorbenzoylamino)-3,4-dihydro-2,2-dimethyl-2H-1-benzopyran-3-ol,
trans-4-(4-Fluorbenzoylmethylamino)-6-trifluormethyl-3,4-dihydro-2,2-dimethyl-2H-1-benzopyran-3-ol,
trans-4-(4-Fluorbenzoylamino)-3,4-dihydro-6-iod-2,2-dimethyl-2H-1-benzopyran-3-ol und
trans-6-Aminocarbonylmethyl-4-(4-fluorbenzoylamino)-3,4-dihydro-2,2-dimethyl-2H-1-benzopyran-3-ol oder einem pharmazeutisch verträglichen Salz davon ausgewählt ist.

7. Verwendung nach Anspruch 4, wobei R₃ und R₄ beide eine Methylgruppe bedeuten.

8. Verwendung nach Anspruch 4, wobei R₅ eine Hydroxygruppe bedeutet und R₆ und R₉ ein Wasserstoffatom bedeuten.

9. Verwendung nach Anspruch 4, wobei X ein Sauerstoffatom bedeutet.

10. Verwendung nach Anspruch 4, wobei Y einen Rest CR₁ bedeutet und R₁ eine Cyano-, Methoxy-, Trifluormethoxygruppe, ein Chloratom, eine Trifluormethyl-, Ethylcarbonyl-, Acetylgruppe, ein Wasserstoffatom, eine Methyl-, Ethyl-, Isopropyl-, tert.-Butyl-, Nitrogruppe, einen Rest C₂F₅, eine Methoxycarbonyl-, Phenylsulfonyl-, Phenylgruppe, ein Fluor-, Iodatom, eine Cyclopentyl-, Aminocarbonylmethyl- und 1-Hydroxyethylgruppe bedeutet und R₂ ein Wasserstoffatom bedeutet.

11. Verwendung nach Anspruch 4, wobei R₁ eine Cyano-, Ethyl- oder Acetylgruppe bedeutet und R₂ ein Wasserstoffatom bedeutet.

12. Verwendung nach Anspruch 4, wobei R₇ eine 2-Fluorphenyl-, 3-Fluorphenyl-, 4-Fluorphenyl-, 2,4-Difluorphenyl- oder 3,4-Difluorphenylgruppe bedeutet.

13. Verwendung nach Anspruch 4, wobei R₈ ein Wasserstoffatom, eine Methyl- oder Ethylgruppe bedeutet.

14. Verwendung nach Anspruch 1 oder 4, wobei die Verbindung in 4S,3R-Konfiguration vorliegt.

15. Verwendung nach Anspruch 14, wobei die Verbindung trans-6-Acetyl-4S-(4-fluorbenzoylamino)-3,4-dihydro-2,2-dimethyl-2H-1-benzopyran-3R-ol ist.

## Revendications

1. Utilisation d'un composé de formule (I) ou d'un de ses sels pharmaceutiquement acceptables ; 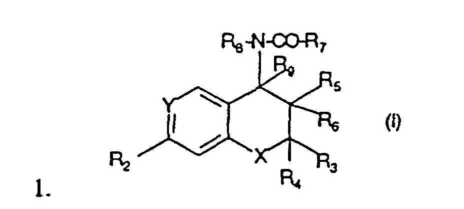 formule dans laquelle :
Y représente N et R₂ représente l'hydrogène, ou bien Y représente un groupe C-R₁
dans lequel :
un des groupes R₁ et R₂ représente l'hydrogène et l'autre est choisi dans le groupe comprenant l'hydrogène, des groupes cycloalkyle en C₃ à C₈, alkyle en C₁ à C₆ facultativement interrompu par l'oxygène ou substitué avec un substituant hydroxy, alkoxy en C₁ à C₆ ou aminocarbonyle substitué, (alkyle en C₁ à C₆) carbonyle, (alkoxy en C₁ à C₆) carbonyle, (alkyle en C₁ à C₆) carbonyloxy, alkoxy en C₁ à C₆, nitro, cyano, halogéno, trifluorométhyle, CF₃S et CF₃-A-, dans lequel A représente un groupe -CF₂-, -CO-, -CH₂- ou CH(OH), trifluorométhoxy, alkylsulfinyle en C₁ à C₆, alkylsulfonyle en C₁ à C₆, alkoxysulfinyle en C₁ à C₆, alkoxysulfonyle en C₁ à C₆, aryle, hétéroaryle, arylcarbonyle, hétéroarylcarbonyle, arylsulfinyle, hétéroarylsulfinyle, arylsulfonyle, hétéroarylsulfonyle dans lequel n'importe quel groupement aromatique est facultativement substitué, (alkyle en C₁ à C₆)carbonylamino, (alkoxy en C₁ à C₆) carbonylamino, (alkyle en C₁ à C₆) thiocarbonyle, (alkoxy en C₁ à C₆) thiocarbonyle, (alkyle en C₁ à C₆) thiocarbonyloxy, 1-mercapto(alkyle en C₂ à C₇), formyle ou aminosulfinyle, aminosulfonyle ou aminocarbonyle, n'importe quel groupement amino étant facultativement substitué avec un ou deux groupes alkyle en C₁ à C₆, ou alkylsulfinylamino en C₁ à C₆, alkylsulfonylamino en C₁ à C₆, alkoxysulfinylamino en C₁ à C₆ ou alkoxysulfonylamino en C₁ à C₆, ou éthylényle substitué en position terminale avec un substituant (alkyle en C₁ à C₆)carbonyle, nitro ou cyano, ou -C(alkyle en C₁ à C₆)NOH ou -C(alkyle en C₁ à C₆)NNH₂, ou bien un des groupes R₁ et R₂ représente un groupe nitro, cyano ou (alkyle en C₁ à C₃)carbonyle et l'autre représente un groupe méthoxy ou amino facultativement substitué avec un ou deux substituants alkyle en C₁ à C₆ ou alcanoyle en C₂ à C₇ ;
un des groupes R₃ et R₄ représente l'hydrogène ou un groupe alkyle en C₁ à C₄ et l'autre représente un groupe alkyle en C₁ à C₄, ou bien R₃ et R₄, conjointement, représentent un groupe polyméthylène en C₂ à C₅ ;
R₅ représente un groupe (alkyle en C₁ à C₆)carbonyloxy, benzoyloxy, ONO₂, benzyloxy, phényloxy ou alkoxy en C₁ à C₆, et R₆ et R₉ représentent l'hydrogène, ou bien R₅ représente un groupe hydroxy et R₆ représente l'hydrogène ou un groupe alkyle en C₁ ou C₂ et R₉ représente l'hydrogène ;
R₇ représente un groupe fluorophényle ;
R₈ représente l'hydrogène ou un groupe alkyle en C₁ à C₆ ;
le groupe R₈-N-CO-R₇ étant en position trans par rapport au groupe R₅ ;
et X représente l'oxygène ou un groupe NR₁₀ dans lequel R₁₀ représente l'hydrogène ou un groupe alkyle en C₁ à C₆ ;
le terme "aryle" désignant un groupe phényle ou naphtyle et le terme "hétéroaryle" désignant un groupe penta- ou hexagonal monocyclique ou nona- ou décagonal bicyclique contenant jusqu'à 3 hétéroatomes qui sont identiques ou différents et qui sont choisis entre l'oxygène, l'azote et le soufre ; et chaque groupe aryle ou hétéroaryle étant facultativement substitué avec jusqu'à trois substituants choisis indépendamment entre des substituants alkyle en C₁ à C₄, alkoxy en C₁ à C₄, halogéno, hydroxy, nitro, cyano et SOₙH dans lequel n a une valeur de 0 à 2 ;
pour la production d'un médicament destiné au traitement et/ou à la prophylaxie d'affections pouvant être traitées ou prévenues avec des agents anticonvulsivants.

2. Utilisation suivant la revendication 1, dans laquelle le composé de formule (I) comprend les variables suivantes, dans lesquelles :
Y représente un groupe C-R₁, dans lequel :
un des groupes R₁ et R₂ représente l'hydrogène et l'autre est choisi dans le groupe consistant en l'hydrogène, des groupes (alkyle en C₁ à C₆) carbonyle, (alkoxy en C₁ à C₆) carbonyle, (alkyle en C₁ à C₆) carbonyloxy, (alkyle en C₁ à C₆) hydroxyméthyle, nitro, cyano, chloro, trifluorométhyle, trifluorométhoxy, alkylsulfinyle en C₁ à C₆, alkylsulfonyle en C₁ à C₆, alkoxysulfinyle en C₁ à C₆, alkoxysulfonyle en C₁ à C₆, (alkyle en C₁ à C₆) carbonylamino, (alkoxy en C₁ à C₆) carbonylamino, (alkyle en C₁ à C₆) thiocarbonyle, (alkoxy en C₁ à C₆) thiocarbonyle, (alkyle en C₁ à C₆) thiocarbonyloxy, 1-mercapto (alkyle en C₂ à C₇), formyle, ou aminosulfinyle, aminosulfonyle ou aminocarbonyle, le groupement amino étant facultativement substitué avec un ou deux groupes alkyle en C₁ à C₆, ou alkylsulfinylamino en C₁ à C₆, alkylsulfonylamino en C₁ à C₆, alkoxysulfinylamino en C₁ à C₆ ou alkoxysulfonylamino en C₁ à C₆, ou éthylényle substitué en position terminale avec un substituant (alkyle en C₁ à C₆) carbonyle, niro ou cyano, ou -C(alkyle en C₁ à C₆) NOH ou -C (alkyle en C₁ à C₆) NNH₂, ou bien un des groupes R₁ et R₂ représente un groupe nitro, cyano ou (alkyle en C₁ à C₃) carbonyle et l'autre représente un groupe méthoxy ou amino facultativement substitué avec un ou deux substituants alkyle en C₁ à C₆ ou alcanoyle en C₂ à C₇ ;
un des groupes R₃ et R₄ représente l'hydrogène ou un groupe alkyle en C₁ à C₄ et l'autre représente un groupe alkyle en C₁ à C₄, ou bien R₃ et R₄, conjointement, représentent un groupe polyméthylène en C₂ à C₅ ;
R₅ représente un groupe hydroxy et R₆ représente l'hydrogène ;
R₇ représente un groupe fluorophényle ;
R₈ représente l'hydrogène ou un groupe alkyle en C₁ à C₆ ; et
R₉ représente l'hydrogène ;
le groupe R₈-N-CO-R₇ étant en position trans par rapport au groupe R₅.

3. Utilisation suivant la revendication 1, dans laquelle le composé est choisi entre les suivants :
trans-6-cyano-3,4-dihydro-2,2-diméthyl-4-(3-fluorobenzoylamino)2H-1-benzopyranne-3-ol,
trans-6-cyano-3,4-dihydro-2,2-diméthyl-4-(2-fluorobenzoylamino)2H-1-benzopyranne-3-ol,
trans-6-trifluorométhoxy-3,4-dihydro-2,2-diméthyl-4-(3-fluorobenzoylamino)2H-1-benzopyranne-3-ol,
trans-6-cyano-4S-(3-fluorobenzoylamino)-3,4-dihydro-2,2-diméthyl-2H-1-benzopyranne-3R-ol,
trans-6-cyano-4R-(3-fluorobenzoylamino)-3,4-dihydro-2,2-diméthyl-2H-1-benzopyranne-3S-ol,
trans-6-cyano-4S-(2-fluorobenzoylamino)-3,4-dihydro-2,2-diméthyl-2H-1-benzopyranne-3R-ol,
trans-6-cyano-4S-(2,4-difluorobenzoylamino)-3,4-dihydro-2,2-diméthyl-2H-1-benzopyranne-3R-ol,
trans-6-acétyl-4-(3-fluorobenzoylamino)-3,4-dihydro-2,2-diméthyl-2H-1-benzopyranne-3-ol,
trans-6-cyano-4-(3,4-difluorobenzoylméthylamino)-3,4-dihydro-2,2-diméthyl-2H-1-benzopyranne-3-ol, et
trans-6-cyano-4S-(3,4-difluorobenzoylamino)-3,4-dihydro-2,2-diméthyl-2H-1-benzopyranne-3R-ol, et leurs sels pharmaceutiquement acceptables.

4. Utilisation suivant la revendication 1, dans laquelle le composé de formule (I) est un composé de formule (Ib) ou un de ses sels pharmaceutiquement acceptables : 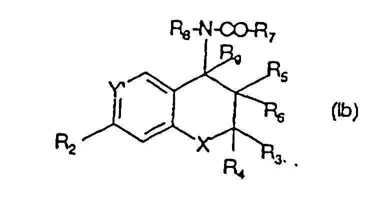 formule dans laquelle les variables R₃, R₄, R₅, R₆, R₇, R₈, R₉ et X répondent aux définitions mentionnées en rapport avec la formule (I) dans la revendication 1 et Y' représente un groupe CR₁ dans lequel R₁ et R₂ représentent l'un et l'autre l'hydrogène ou bien un des groupes R₁ et R₂ représente un groupe trifluorométhoxy, alkyle en C₁ à C₆ interrompu par l'oxygène ou substitué avec un substituant hydroxy, alkoxy en C₁ à C₆ ou aminocarbonyle substitué, CF₃A- (dans lequel A représente un groupe -CF₂-, -CO-, -CH₂ ou CH(OH), arylsulfonyle, aryle, cycloalkyle en C₃ à C₈, alkoxy en C₁ à C₆, hétéroaryle, arylcarbonyle, hétéroarylcarbonyle, arylsulfinyle, hétéro-arylsulfinyle, hétéroarylsulfonyle, dans lequel n'importe quel groupement aromatique est facultativement substitué, et l'autre représente l'hydrogène.

5. Utilisation suivant la revendication 4, dans laquelle le composé est choisi entre les suivants :
trans-3,4-dihydro-2,2-diméthyl-4-(4-fluorobenzoylamino)-2H-1-benzopyranne-3-ol,
trans-4-(4-fluorobenzoylamino)-3,4-dihydro-2,2,6-triméthyl-2H-1-benzopyranne-3-ol,
trans-6-éthyl-4-(4-fluorobenzoylamino)-3,4-dihydro-2,2-diméthyl-2H-1-benzopyranne-3-ol,
trans-6-éthyl-4-(4-fluorobenzoyléthylamino)-3,4-dihydro-2,2-diméthyl-2H-1-benzopyranne-3-ol,
trans-6-éthyl-4-(4-fluorobenzoylméthylamino)-3,4-dihydro-2,2-diméthyl-2H-1-benzopyranne-3-ol,
trans-4-(4-fluorobenzoylamino)-6-pentafluoréthyl-3,4-dihydro-2,2-diméthyl-2H-1-benzopyranne-3-ol,
trans-4-(4-fluorobenzoylamino)-3,4-dihydro-2,2-diméthyl-6-phénylsulfonyl-2H-1-benzopyranne-3-ol,
trans-4-(4-fluorobenzoylamino)-3,4-dihydro-2,2-diméthyl-6-phényl-2H-1-benzopyranne-3-ol,
trans-6-éthyl-4S-(4-fluorobenzoylamino)-3,4-dihydro-2,2-diméthyl-2H-1-benzopyranne-3R-ol,
trans-4R-(4-fluorobenzoylamino)-3,4-dihydro-6-(1-hydroxyéthyl)-2,2-diméthyl-2H-1-benzopyranne-3S-ol,
trans-4S-(4-fluorobenzoylamino)-3,4-dihydro-6-(1-hydroxyéthyl)-2,2-diméthyl-2H-1-benzopyranne-3R-ol,
trans-4-(4-fluorobenzoylméthylamino)-3,4-dihydro-2,2-diméthyl-2H-1-benzopyranne-3-ol,
trans-6-tertio-butyl-4-(4-fluorobenzoylamino) -3,4-dihydro-2,2-diméthyl-2H-1-benzopyranne-3-ol,
trans-4-(4-fluorobenzoylamino)-3,4-dihydro-2,2-diméthyl-6-cyclopentyl-2H-1-benzopyranne-3-ol, et
trans-4-(4-fluorobenzoylamino)-3,4-dihydro-6-méthoxy-2,2-diméthyl-2H-1-benzopyranne-3-ol, et leurs sels pharmaceutiquement acceptables.

6. Utilisation suivant la revendication 1, dans laquelle le composé est choisi entre les suivants :
trans-6-cyano-3,4-dihydro-2,2-diméthyl-4-(4-fluorobenzoylamino)2H-1-benzopyranne-3-ol,
trans-6-chloro-3,4-dihydro-2,2-diméthyl-4-(4-fluorobenzoylamino)2H-1-benzopyranne-3-ol,
trans-6-trifluorométhyl-4-(4-fluorobenzoylamino)-3,4-dihydro-2,2-diméthyl-2H-1-benzopyranne-3-ol,
trans-6-cyano-4-(4-fluorobenzoylméthylamino)-3,4-dihydro-2,2-diméthyl-2H-1-benzopyranne-3-ol,
trans-6-éthylcarbonyl-3,4-dihydro-2,2-diméthyl-4-(4-fluorobenzoylamino)2H-1-benzopyranne-3-ol,
trans-6-éthylcarbonyl-3,4-dihydro-2,2-diméthyl-4-(4-fluorobenzoylméthylamino)2H-1-benzopyranne-3-ol,
trans-6-acétyl-4-(4-fluorobenzoylméthylamino)-3,4-dihydro-2,2-diméthyl-2H-1-benzopyranne-3-ol,
trans-6-cyano-3,4-dihydro-4-(4-fluorobenzoyléthylamino)-2,2-diméthyl-2H-1-benzopyranne-3-ol,
trans-4-(4-fluorobenzoylamino)-3,4-dihydro-2,2-diméthyl-7-nitro-2H-1-benzopyranne-3-ol,
trans-6-acétyl-4R-(4-fluorobenzoylamino)-3,4-dihydro-2,2-diméthyl-2H-1-benzopyranne-3S-ol,
trans-6-acétyl-4S-(4-fluorobenzoylamino)-3,4-dihydro-2,2-diméthyl-2H-1-benzopyranne-3R-ol,
trans-6-cyano-4S-(4-fluorobenzoylamino)-3,4-dihydro-2,2-diméthyl-2H-1-benzopyranne-3R-ol,
trans-6-cyano-4-(4-fluorobenzoylamino)-3,4-dihydro-2,2,3-triméthyl-2H-1-benzopyranne-3-ol,
trans-4-(4-fluorobenzoylamino)-3,4-dihydro-2,2-diméthyl-6-nitro-2H-1-benzopyranne-3-ol,
trans-4-(4-fluorobenzoylamino)-3,4-dihydro-6-méthoxycarbonyl-2,2-diméthyl-2H-1-benzopyranne-3-ol,
trans-6-fluoro-4-(4-fluorobenzoylamino)-3,4-dihydro-2,2-diméthyl-2H-1-benzopyranne-3-ol,
trans-4-(4-fluorobenzoylméthylamino)-6-trifluorométhyl-3,4-dihydro-2,2-diméthyl-2H-1-benzopyranne-3-ol,
trans-4-(4-fluorobenzoylamino)-3,4-dihydro-6-iodo-2,2-diméthyl-2H-1-benzopyranne-3-ol, et
trans-6-aminocarbonylméthyl-4-(4-fluorobenzoylamino)-3,4-dihydro-2,2-diméthyl-2H-1-benzopyranne-3-ol, et leurs sels pharmaceutiquement acceptables.

7. Utilisation suivant la revendication 4, dans laquelle R₃ et R₄ représentent l'un et l'autre un groupe méthyle.

8. Utilisation suivant la revendication 4, dans laquelle R₅ représente un groupe hydroxy et R₆ et R₉ représentent l'hydrogène.

9. Utilisation suivant la revendication 4, dans laquelle X représente l'oxygène.

10. Utilisation suivant la revendication 4, dans laquelle Y représente un groupe CR₁ et R₁ représente un groupe cyano, méthoxy, trifluorométhoxy, chloro, trifluorométhyle, éthylcarbonyle, acétyle, hydrogène, méthyle, éthyle, isopropyle, tertio-butyle, nitro, C₂F₅, méthoxycarbonyle, phénylsulfonyle, phényle, fluoro, iodo, cyclopentyle, aminocarbonylméthyle et 1-hydroxyéthyle et R₂ représente l'hydrogène.

11. Utilisation suivant la revendication 4, dans laquelle R₁ représente un groupe cyano, éthyle ou acétyle et R₂ représente l'hydrogène.

12. Utilisation suivant la revendication 4, dans laquelle R₇ représente un groupe 2-fluorophényle, 3-fluorophényle, 4-fluorophényle, 2,4-difluorophényle ou 3,4-difluorophényle.

13. Utilisation suivant la revendication 4, dans laquelle R₈ représente l'hydrogène, un groupe méthyle ou éthyle.

14. Utilisation suivant la revendication 1 ou 4, dans laquelle le composé a la configuration 4S, 3R.

15. Utilisation suivant la revendication 14, dans laquelle le composé est le trans-6-acétyl-4S-(4-fluorobenzoylamino)-3,4-dihydro-2,2-diméthyl-2H-1-benzopyranne-3R-ol.
